# EUROPEAN PATENT APPLICATION

(11) **EP 0 974 349 A1**
(43) Date of publication of application: **26.01.2000**
(21) Application number: 98901566.4
(22) Date of filing: 09.02.1998
(51) Int. Cl.: A61K 31/19, A61K 31/365, A61K 35/78, A23L 1/30, A23L 1/16, A23L 1/22

(54) **ATHLETIC ENDURANCE INCREASING AGENT**

(30) Priority: 13.02.1997 JP 2891497
(71) Applicant: Nippon Shinyaku Co., Ltd., Kyoto-shi Kyoto 601-8550 (JP)
(72) Inventor: FUSHIKI, Tohru, Otsu-shi Shiga 520-0113 (JP); ISHIHARA, Kengo, Otsu-shi Shiga 520-0113 (JP); ANNO, Takahiko, Otsu-shi Shiga 520-0112 (JP); TOMI, Hironori, Otsu-shi Shiga 520-2153 (JP)
(74) Representative: Strych, Werner Maximilian Josef, Dr.
(86) International application number: JP9800533
(87) International publication number: WO9835664

(57) **Abstract**

A new use of a hydroxycitric acid and derivatives thereof; and a new method of utilizing a garcinia extract containing any of the hydroxycitric acid and derivatives. The athletic endurance reinforcing agent is characterized by containing as the active ingredient (-)-hydroxycitric acid, its lactone, or a salt of either.

## Description

### TECHNICAL FIELD

This invention relates to an exercise endurance enhancer characterized by its comprising (-)-hydroxycitric acid (HCA) or a lactone form thereof, or a salt thereof, (all of which are collectively referred to herein as "hydroxycitric acid compound").

The term "exercise endurance" is used in this specification to mean the ability to continuously perform a given physical work.

### BACKGROUND ART

The hydroxycitric acid compound is known, at least ïn part, by its presence in the pericarps of trees of the Garcinia genus which are evergreens distributed in tropical districts, and this compound is known to inhibit ATP citrate lyase, one of the enzymes involved in the pathway synthesizing lipids from citric acid, to suppress increase in body adipose, thus being useful for the therapy of obesity (e.g. US Patent 3,764,692). Actually, garcinia extracts containing the hydroxycitric acid compound are in popular use as health foods for the therapy of obesity.

Meanwhile, it is known that the hydroxycitric acid compound suppresses lipid synthesis by inhibiting ATP citrate lyase and, hence, promotes glycogen synthesis as much to cause an accumulation of glycogen in the liver [e.g. Federation Proceedings, Vol. 44, No. 1, 139-144 (1985), Life Sciences, Vol. 53, No. 24, 1833-1845 (1993)]. However, no correlation has been found between accumulated liver glycogen and exercise endurance [e.g. Dohm G. L. et al., J. Appl. Physiol., 61, 1363-1368 (1986), 55, 830-833 (1983)].

### DISCLOSURE OF THE INVENTION

The object of this invention is to provide a novel use of the hydroxycitric acid compound and a novel method for use of a garcinia extract containing the hydroxycitric acid compound.

In the course of intensive research, the inventors of this invention discovered by chance that the hydroxycitric acid compound having a body adipose increase-inhibitory action has an exercise endurance-enhancing action as well and have developed this instant invention. Admitting that the increase in body adipose is inhibited, it is logical to suppose that the sources of energy for the muscles are curtailed to affect exercise endurance adversely. Therefore, it was a surprise that the hydroxycitric acid compound showed an exercise endurance enhancing action.

This invention, therefore, can be categorized as an exercise endurance enhancer characterized by its comprising (-)-hydroxycitric acid or a lactone form thereof or a salt thereof as active ingredient. Thus, a composition characterized by comprising (-)-hydroxycitric acid or a lactone form thereof, or a salt thereof, as active ingredient can be used for the purpose of enhancing exercise endurance.

This invention may also be categorized as an exercise endurance enhancer characterized by comprising (-)-hydroxycitric acid or a lactone form thereof, or a salt thereof, which is available from an extract of the garcinia pericarp which contains (-)-hydroxycitric acid or a lactone thereof, or a salt thereof, as active ingredient.

The preferred hydroxycitric acid compound includes (-)-hydroxycitric acid and water-soluble salts of (-)-hydroxycitric acid. Therefore, the exercise endurance enhancer of this invention (hereinafter referred to as the enhancer of the invention) is preferably an enhancer containing (-)-hydroxycitric acid or a water-soluble salt of (-)-hydroxycitric acid as active ingredient. Particularly preferred is (-)-hydroxycitric acid which has not formed a salt.

The salt of hydroxycitric acid compound includes but is not limited to alkali metal salts such as sodium salt, potassium salt, etc., alkaline earth metal salts such as magnesium salt, calcium salt, barium salt, etc., ammonium salts, and ethylenediamine salt. In particular, a water-soluble salt of (-)-hydroxycitric acid is preferred as mentioned above, and the sodium salt or the potassium salt can be mentioned as the preferred species of the salt.

While the hydroxycitric acid compound can be produced by chemical synthesis (e.g. Z. Physiol. Chem., 269, 33 (1941)), it is common practice to extract it from the garcinia pericarp containing the hydroxycitric acid compound in accordance with the known procedure. For example, an extract containing the hydroxycitric acid compound can be obtained by extracting garcinia pericarps containing the hydroxycitric acid compound with water or alcohol in the conventional manner. This extract can be concentrated to any desired hydroxycitric acid compound level by the routine procedure (e.g. Phytochemistry, 4, 619-625 (1965)) and, whether in the extraction process or after extraction, (-)-hydroxycitric acid etc. can be caused to form suitable salts. Furthermore, the method described in USP 5,536,516 can be used to provide an extract rich in (-)-hydroxycitric acid with the proportion of the components (e.g. sugars) other than said hydroxycitric acid compound having been curtailed. The lactone form of (-)-hydroxycitric acid or its salt can be prepared in accordance with the method of R. P. Singh [Biol. Memoirs, 21, 27-33 (1995)], for instance. Moreover, the above extract can be processed into powders with the aid of a suitable excipient (e.g. dextrin, gum Arabic, modified starch) by the routine spray-drying or spray-granulation technique.

The garcinia containing the hydroxycitric acid compound includes but is not limited toGarcinia cambogia, Garcinia indica, and Garcinia atroviridis.

Extracts from those sources are known, and such known garcinia extracts as such can be used as the enhancer of the invention. For example, Garcinia Extract S (tradename, manufactured by Nippon Shinyaku Co., Ltd.), Garcinia Powder S (tradename, manufactured by Nippon Shinyaku Co., Ltd.), Citrin (tradename, manufactured by Sabinsa (USA)) and Citrimax (tradename, manufactured by Interhealth (USA), all of which are commercially available, can each be used directly as the enhancer of the invention.

Furthermore, the above known extracts can be formulated with a suitable excipient and processed into the enhancer of the invention or a food containing the enhancer of the invention (hereinafter referred to as the food of the invention). The excipient mentioned above includes but is not limited to food ingredients and food additives, such as the carbohydrate (sugars and sugar alcohols), sweetener, acidulant, antioxidant, shelf-life extender, and preservative. Among them, the carbohydrate, sweetener and acidulant can be judiciously formulated each within the range of 0.01-90 weight %, preferably 0.1-70 weight %. The antioxidant, shelf-life extender and preservative can be judiciously formulated each within the range of 0.001-10 weight %, preferably 0.01-1 weight %. Aside from the above, vitamins, amino acids, minerals, a thickener-stabilizer, colors, diet fiber, a flavor, an emulsifier, a pH control agent, etc. can be formulated in suitable amounts.

The carbohydrate mentioned above specifically includes but is not limited to starch, starch syrup, saccharified reducing starch, erythritol, sorbose, mannitol, lactulose, lactitol, palatinit, maltitol, xylobiose, fructofuranosylnystose, (α , β )-cyclodextrin, maltosyl-β -cyclodextrin, sorbitol, theande-oligo, maltotetraitol, sucrose, glucose, fructose, lactose, maltose, xylose, trehalose, galactose, xylitol, palatinose, fructo-oligosaccharide and lactofructo-oligosaccharide.

The sweetener specifically includes but is not limited to aspartame, sweet hydrangea leaf extract, licorice extract, D-xylose, disodium glycyrrhizinate, enzymatically processed licorice, saccharin, saccharin sodium, stevia extract, stevia powder, L-sorbose, thaumatin, tian ridng cha extract, L-fucose, Rakanka extract, L-rhamnose, D-ribose, miraculin, curcurin, monellin, and dihydrochalcone.

The acidulant specifically includes but is not limited to adipic acid, itaconic acid, citric acid, trisodium citrate, gluconic acid, α -ketoglutaric acid, succinic acid, monosodium succinate, disodium succinate, sodium acetate, L-tartaric acid, DL-tartaric acid, sodium L-tartrate, sodium DL-tartrate, lactic acid, sodium lactate, glacial acetic acid, phytic acid, fumaric acid, monosodium fumarate, DL-malic acid, sodium DL-malate and phosphoric acid.

The antioxidant specifically includes but is not limited to L-ascorbic acid, dl-α -tocopherol, propyl gallate, sage extract, extracted tocopherol, catechin, sesame oil extract, hesperetin, rosemary extract, butylhydroxyanisole and dibutylhydroxytoluene.

The shelf-life extender specifically includes but is not limited to acetic acid, sodium acetate, glycine, mulberry extract, lysozyme, clove extract, tea extract, perilla extract and glycerin fatty acid esters.

The preservative specifically includes but is not limited to benzoic acid, sodium benzoate, sorbic acid, potassium sorbate, sodium dehydroacetate, ε -polylysine, pectin digest, milt protein, ethyl p-hydroxybenzoate.

The vitamin includes but is not limited to vitamin A, vitamin B₁, vitamin B₂, vitamin B₆, vitamin B_{1 2}, vitamin C, vitamin E, vitamin D, nicotinic acid and pantothenic acid.

The amino acid specifically includes but is not limited to L-asparagine, L-aspartic acid, L-isoleucine, L-cystine, L-cysteine hydrochloride, L-serine, L-thyrosine, DL-tryptophan, L-tryptophan, L-threonine, L-valine, L-methionine, L-lysine, L-leucine and L-carnitine.

The mineral specifically includes but is not limited to calcium carbonate, baked calcium, unbaked calcium, iron citrate, ferrous gluconate, zinc gluconate and copper gluconate.

The thickener-stabilizer includes but is not limited to sodium alginate, carboxymethylcellulose sodium, methylcellulose, poly(sodium acrylate), tamarindseed gum, carrageenan, xanthan gum, gellan gum, pectin, chitin, chitosan, pullulan and aloe vera extract.

Furthermore, the enhancer of the invention can be provided in combination with various health food materials. The health food materials include but are not limited to red pepper (capsaicin), Acanthopanacis Senticosi Radix, ginseng, chlorella, avocado, snapping turtle, bracket fungi of the genus Fomes, oyster shell, Eucommia ulmoides, deep-sea shark, propolis, spirulina, low striped bamboo, Houttuynia cordata, Japanese yew leaf, plum, gymnema, laminaria root, corb shell, germs, alfalpha, lamprey, water oat, Angelica keiskei (ashitaba) and cordyceps, inclusive of extracts thereof, royal jelly, nucleic acids, DHA, lecithin, EPA, gamma-linolenic acid, and saponin.

The food according to this invention includes but is not limited to sweetmeats such as cookies, candies, jellies, gummy candies, sherbets, cakes, buns, chewing gums, ice cream, cream puffs, waffles, rice crackers, sweetmeat tablets, etc., beverages such as fruit juice-containing drinks, non-fruit juice drinks, carbonated drinks, lactic acid bacteria-containing drinks, coffee, tea, etc., solid, gel, liquid or combination type sport foods and sport drinks, health foods in the form of capsules, tablets or powders, seasonings such as dressings, soy sauces, curry powder premix, curry roux, etc., and other foods such as noodles, bakery foods, cereal foods, retort foods, and so on.

The daily intake, in adult humans, of the hydroxycitric acid compound from the enhancer of the invention or the food of the invention is dependent on each individual and the timing of ingestion but may judiciously be 10 mg-20 g, preferably 50 mg-2 g, more preferably 250 mg-1.5 g. The effect of this invention may not be materialized when the amount of intake is less than 0.01 g or more than 20 g.

The timing of ingestion of the enhancer of the invention or the food of the invention is not particularly restricted but is preferably during the fast 30 minutes to 1 hour before meal. It is also preferable to ingest the enhancer or food immediately after an exercise.

Where necessary, the enhancer of the invention and the food of the invention may be ingested in 2⁻6 divided doses daily.

### BEST MODE FOR CARRYING OUT THE INVENTION

The following examples and test examples illustrate this invention in further detail.

### Example 1 Tablets containing the hydroxycitric acid compound

Tablets conforming to the recipe given in Table 1 below were prepared by the well-established direct compression method. Here, % is by weight.

**[Table 1]**

| | |
|---|---|
| Garcinia powder S (mfg. by Nippon Shinyaku Co., Ltd.) | 35.5% |
| Sunmalt-S (mfg. by Hayashibara Shoji K.K.) | 31.5% |
| Palatinit (mfg. by Mitsui Seito Co. Ltd.) | 8.0% |
| Erythritol, granular (mfg. by Nikken Chemicals Co., Ltd.) | 9.0% |
| Fermented milk powder (mfg. by Taiyo Koryo Co. Ltd.) | 7.0% |
| Yogurt flavor (mfg. by Shiono Koryo Co., Ltd.) | 0.8% |
| Fruit mix flavor (mfg. by Shiono Koryo Co., Ltd.) | 0.4% |
| Sucrose fatty acid ester DK Ester F-20W (mfg. by Dai-Ichi Kogyo Seiyaku Co., Ltd.) | 3.15% |
| Vitamin C powder (mfg. by Takeda Chemical Ind., Ltd.) | 2.5% |
| Vitamin B₁ powder (mfg. by Takeda Chemical Ind., Ltd.) | 0.03% |
| Vitamin B₂ 〈10% powder〉 (mfg. by Takeda Chemical Ind., Ltd.) | 0.8% |
| Vitamin B₆ powder (mfg. by Takeda Chemical Ind., Ltd.) | 0.08% |
| Vitamin B_{1 2} 〈0.1% powder〉 (mfg. by Takeda Chemical Ind., Ltd.) | 0.14% |

### Example 2 A drink containing the hydroxycitric acid compound

A nonalcoholic beverage conforming to the recipe given in Table 2 below was prepared by the routine procedure. Here, % is by weight.

**[Table 2]**

| | |
|---|---|
| Garcinia extract S (mfg. by Nippon Shinyaku Co. Ltd.) | 1.0% |
| Mabit syrup (mfg. by Hayashibara Shoji K.K.) | 5.0% |
| Erythritol (mfg. by Nikken Chemicals Co., Ltd.) | 4.0% |
| Mayoligo 55P (mfg. by Yakult Pharmaceutical Co. Ltd.) | 1.5% |
| Pine fiber (mfg. by Matsutani Chemical Industry, Ltd.) | 2.5% |
| Trisodium citrate (mfg. by Tanabe Seiyaku Co., Ltd.) | 0.3% |
| Na carbonate 〈granular ash〉 (mfg. Asahi Ryushi Co., Ltd.) | 0.1% |
| Yogurt essence SW-3887 (mfg. by Shiono Koryo Co. Ltd.) | 0.02% |
| Lemon lime flavor (mfg. by Shiono Koryo Co. Ltd.) | 0.02% |
| Lemon essence (mfg. by Shiono Koryo Co. Ltd.) | 0.005% |
| Vitamin C-Na (mfg. by Takeda Chemical Ind., Ltd.) | 0.1% |

### Test Example 1 Exercise endurance enhancing effect in mice

In accordance with the experimental protocol described in the literature (J. Appl. Physiol, 81(4) 1843-1849 (1996)), the exercise endurance enhancing effect of the hydroxycitric acid compound was evaluated by measuring the swimming time of mice.

Six-week-old male ddY mice (ca 30 g) were used in 2 groups of 6 individuals and the animals were allowed a solid diet (MF, Oriental Yeast Co., Ltd.) and water ad libitum throughout the trial period. For use as the test sample, Garcinia Extract S (tradename, Nippon Shinyaku Co. Ltd.) was diluted with water to a 5 weight % concentration of free (-)-hydroxycitric acid (HCA) (hereinafter referred to as the HCA solution). The forced-swimming experiment (preliminary swimming and test swimming sessions) was performed in a specially desired running-water pool (sized 90 cm long x 45 cm wide x 45 cm high; depth of water 38 cm) controlled at a flow rate of 8 L/min. and a water temperature of 34°C. The lighting schedule for the animal room during the trial period was ON from 6:00 p.m. to 6:00 a.m. and OFF from 6:00 a.m. to 6:00 p.m.

First, a preliminary swimming session without administration of the sample was started at 1:00 p.m. 2 days preceding the test swimming session to measure the maximum swimming time. On the day immediately preceding the test swimming session, 100 µ L of distilled water was administered orally to a control group and 100 µ L of HCA solution (HCA 5 mg) to an HCA-dosed group, during the hour from 5:00 p.m. to 6:00 p.m. (Day 0). On the following day, the test swimming session was started at 1:00 p.m. and the animal was forced to swim up to the exhaust limit. Just as on the previous day, distilled water or the HCA solution was administered orally during the hour from 5:00 p.m. to 6:00, p.m. (Day 1). The above procedure was repeated on the following day (Day 2), and the maximum swimming time data were generated on the third day starting at 1:00, p.m. (Day 3).

The results are shown in Table 3.

**Table 3**

| | Max. preliminary swimming time (before administration) | Maximum swimming time at day 3 (after administration) |
|---|---|---|
| control group | 33.19 ± 18.06 | 40.51 ± 22.08 |
| HCA group | 33.95 ± 12.67 | 64.75 ± 20.87* |

| | | |
|---|---|---|
| * : p<0.03 from control, Mean± standard deviation; unit (min) | | |

It will be apparent from Table 3 that a significant enhancement of execise (swimming) endurance capacity was obtained in the HCA-dosed group.

### Test Example 2 Exercise endurance enhancing effect in humans

As a parameter of exercise endurance, the maximum oxygen uptake capacity is known. This parameter represents the aerobic ATP generating activity (McArdle, W. D., Katch, V. I. and Pechar, G. S., Med. Sci. Sports, 5, 156-160, (1973)) and an increase in this maximum oxygen uptake capacity represents an increase in exercise endurance. The exercise endurance enhancing effect was evaluated by measuring this maximum oxygen uptake capacity.

In the experiment, a subject (42-year-old, male, body weight 95 kg) received 4 tables each containing 62.5 mg of HCA (tradename: Fruity Garcinia; distributed by Royal K. K.) 3 times daily before each meal (daily HCA intake 750 mg) for 7 days. The maximum oxygen uptake capacity was measured before administration (day 0), day 7 after start of administration and day 2 after completion of administration (day 9 after start of administration).

Measurement of the maximum oxygen uptake capacity was made by the method of Miyashita et al. using a solenoid-brake bicycle ergometer [Cycle Exa EP351, Matsushita Electric Works, Ltd.] [Miyashita, M., Muto, Y., Yoshioka, N., Sadamoto, T.: J. J. Sport Sci., 2, 912-916 (1983)].

The results are presented in Table 4.

**Table 4**

| | Maximum oxygen uptake capacity (ml/kg/min) |
|---|---|
| Before administration | 28.7 |
| Day 7 after start of administration | 34.8 |
| Day 2 after completion of administration | 28.8 |

It will be apparent from Table 4 that administration of HCA caused a definite enhancement of exercise endurance.

### EFFECTS OF THE INVENTION

The enhancer of the invention is sufficiently effective in enhancing exercise endurance. Moreover, although (-)-hydroxycitric acid not in the form of a salt is reportedly low in activity because it assumes a lactone form, even this (-)-hydroxycitric acid was found to have a sufficient exercise endurance-enhancing action.

## Claims

1. An exercise endurance enhancer characterized by comprising (-)-hydroxycitric acid or a lactone form thereof, or a salt thereof, as active ingredient.

2. An exercise endurance enhancer characterized by comprising (-)-hydroxycitric acid or a water-soluble salt of (-)-hydroxycitric acid as active ingredient.

3. An exercise endurance enhancer characterized by comprising (-)-hydroxycitric acid or a lactone form thereof, or a salt thereof, as originating from an extract of garcinia pericarps containing (-)-hydroxycitric acid or a lactone form thereof, or a salt thereof, as active ingredient. .

4. An exercise endurance enhancer characterized by comprising (-)-hydroxycitric acid or a water-soluble salt of (-)-hydroxycitric acid as originating from an extract of garcinia pericarps containing (-)-hydroxycitric acid or a lactone form thereof, or a salt thereof, as active ingredient.

5. The exercise endurance enhancer defined in either Claim 3 or Claim 4 wherein the garcinia is Garcinia cambogia, Garcinia indica or Garcinia atroviridis.

6. The exercise endurance enhancer defined in either Claim 2 or Claim 4 wherein the water-soluble salt of (-)-hydroxycitric acid is the sodium salt or the potassium salt.

7. A food containing the exercise endurance enhancer defined in any of Claims 1 through 6.

8. The food defined in Claim 7 which is a confection, a drink, a sport food, a sport drink, a health food, a seasoning, a food ingredient, a noodle, a bakery food, a cereal food or a retort food.
